# EUROPEAN PATENT APPLICATION

(11) **EP 2 942 009 A1**
(43) Date of publication of application: **11.11.2015**
(21) Application number: 13883056.7
(22) Date of filing: 26.06.2013
(51) Int. Cl.: A61B 5/12, H04R 25/00

(54) **DEVICE FOR HEARING DIAGNOSIS AND TREATMENT**

(30) Priority: 27.04.2013 CN 201310158407
(71) Applicant: Jiangsu Betterlife Medical Co., Ltd., Jiangsu 215500 (CN)
(72) Inventor: ZHAO, Bingbing, Jiangsu 215500 (CN); ZHAO, Yong David, Jiangsu 215500 (CN); ZHAO, Jennifer Jinping, Jiangsu 215500 (CN)
(74) Representative: Becker Kurig Straus
(86) International application number: PCT/CN2013/000759
(87) International publication number: WO 2014/172812

(57) **Abstract**

Disclosed is a hearing diagnosis and treatment device, comprising at least an all-digital audio and tone signal synthesis generator for generating various audio and tone signals meeting hearing diagnosis and treatment conditions, a digital information management and transmission module connected with the all-digital audio and tone signal synthesis generator, and a tinnitus diagnosis and treatment unit and a hearing-aid diagnosis and treatment unit which are connected with the all-digital audio and tone signal synthesis generator. On the basis of the above, the hearing diagnosis and treatment device of the present invention integrates the tinnitus diagnosis and treatment unit and the hearing-aid diagnosis and treatment unit which share the same all-digital audio and tone signal synthesis generator, so the device is simplified, saves cost, is conveniently operable and portable and widely applicable, and provides patients with hearing problems with individual and specific diagnosis and treatment.

## Description

### FIELD OF THE INVENTION

The present invention relates to the field of hearing diagnosis and treatment, in particular to a hearing diagnosis and treatment device on a basis of an all-digital audio and tone signal synthesis generator module.

### BACKGROUND OF THE INVENTION

Hearing test is a technology of judging whether the hearing function is normal through external sound stimulation. It can be used to diagnose and identify hearing disorder or hearing loss. To better judge the degree of hearing loss and fit the hearing aid, a hearing test device is needed.

The hearing test device is a customary device for testing hearing and widely applied in the clinical medicine. Different from other tests, strict requirements for the test device and special requirements, in particular the environmental noises, for the environment for the hearing test are imposed to ensure accurate and reliable hearing test result. Environmental noises exceeding a certain limit can cause bad effects on the test results. The accuracy and reliability of the test result can be ensured only when the environmental noises meet a certain requirements. Moreover, the hearing test process is complicated. Clinically, the simulated objective test method is employed, which is also disadvantaged in complicated data conversion process and difficulty in ensuring the accuracy. Hearing test comprises objective hearing test (for example, acoustic impedance OAE test, auditory brainstem response (ABR) test, auditory steady state response (ASSR) test), and\or objective hearing test (for example, pure tone hearing test). Those hearing test devices, which are different in principles and properties, are mutually independent and are not mutually connected; employing self-prepared external single tone stimulating sound sources. However, whether the hearing level obtained by testing using those single tone stimulating sound sources can represent the sense level of the auditory nerve to various compound sound signals in real life is always doubted. Particularly, a deaf child can hear, but it does not mean that he/she can hear clearly and understand. In comparison with single, bald tones, some compound tones or languages that children are fond of can better stimulate their auditory nerves (for example, children are born to be very sensitive to some minimal sounds, music, and words like Mum and Papa), so the auditory level thereof can be more accurately reflected, the fitting and debugging of the hearing-aid be more effectively instructed, and the hearing-aid's effect of assisting hearing recovery be optimized. Therefore, it is very necessary to perform a comprehensive assessment on the hearing test results of various hearing test devices, expand the stimulating sound for testing from single tone to compound tones, and event simulated animal sound and human speeches.

Tinnitus is a hearing disorder, which causes a patient to feel certain or several sounds in his/her ears or head on condition of no external environmental sounds. The majority of subjective tinnitus is untreatable yet. Various drug, masking, acclimatization, acupuncture and moxibustion, electric/magnetic stimulation, ultrasonic, high-pressure oxygen, hypnosis, yoga and psychological therapies all have certain effects on some tinnitus patients, of which the masking therapy and acclimatization therapy are a kind of physiological acoustic treatment methods, needing special tinnitus treatment devices. Those two methods are simple and safe, do not generate obvious side effects, and are customary methods for treating tinnitus.

At present, the masking therapy usually refers to a method of letting a patient to hear narrow-band noises with a frequency close to that of the tinnitus sound regularly, and then the noises mask the tinnitus sound of the patient. The acclimatization therapy refers to a method of letting a patient to hear some wide-frequency relaxative natural sounds, and then the patient is gradually used to and adapted to the tinnitus sound.

At present, the noise frequencies and loudness played by special tinnitus treatment device available on the market are set by a doctor for a patient in advance through a special instrument. However, the main frequency and\loudness and\ tone and \or melody may change irregularly. Those tinnitus treatment devices cannot generate digital signals of sounds from single tones such as the simple pure tone and noises to compound tones such as the complicated compound sound, simulated animal sound, simulated human speeches; the frequencies, loudness, tone, melody and rhythm cannot be adjusted randomly. Those devices cannot provide individual and specific tinnitus masking therapy or tinnitus acclimatization therapy and thus, the tinnitus treatment effects are undesirable. The special tinnitus treatment devices available on the market cannot generate various sounds meeting the needs of the clinical chemical diagnosis.

As mentioned above, the hearing test device is needed during the hearing test, and the special tinnitus treatment device is needed during the tinnitus diagnosis. The separate devices are very complicated, and increase corresponding cost. Besides, in the hearing-aid fitting process and other hearing test and hearing diagnosis and treatment process, generation, recording and storage of various sounds are involved.

### SUMMARY OF THE INVENTION

The present invention mainly solves the technical problem of providing a hearing diagnosis and treatment device, which provides an audio and tone signal generator capable of generating various sounds and shares the sound sources and integration through a tinnitus diagnosis and treatment unit and a hearing-aid diagnosis and treatment unit. The hearing diagnosis and treatment device solves the problems of complicated separate devices and high cost during hearing-aid diagnosis and tinnitus diagnosis and treatment in the prior art, imposes no requirement on the environmental noises, avoids errors caused by the traditional input, and provides individual and specific diagnosis and treatment formulas.

To solve the above technical problem, the present invention employs a technical solution: Disclosed is a hearing diagnosis and treatment device, comprising at least an all-digital audio and tone signal synthesis generator for generating various audio and tone signals meeting hearing diagnosis and treatment conditions; a digital information management and transmission module connected with the all-digital audio and tone signal synthesis generator; and also comprising a tinnitus diagnosis and treatment unit and a hearing-aid diagnosis and treatment unit which are directly connected with the all-digital audio and tone signal synthesis generator.

In a preferably embodiment of the present invention, the all-digital audio and tone signal synthesis generator can generate acoustic digital signals from single tone to compound tone such as the pure tone and \or noises and\or compound sound and \or simulated animal sound and \or simulated human speech, and can randomly adjust the frequency and\ or loudness and\or tone and \or melody and \or rhythm; the tinnitus diagnosis and treatment unit and the hearing-aid diagnosis and treatment unit share the same all-digital audio and tone signal synthesis generator.

In a preferably embodiment of the present invention, the hearing diagnosis and treatment device also comprises an otoscope digital image detecting module for detecting the auditory meatus and tympanic membrane of a patient and outputting detection results in digital and image form to the digital information management and transmission module or a printer.

In a preferably embodiment of the present invention, the hearing diagnosis and treatment device is internally provided with a wireless transmission module, capable of exchanging information such as the digital images of the auditory meatus and tympanic membrane, the test result and treatment prescriptions of the tinnitus diagnosis and treatment unit, the hearing test and auditory assessment results and hearing compensation and optimizing prescriptions of the hearing-aid diagnosis and treatment unit in the digital information management and transmission module with the information in a remote database or storing the information.

In a preferably embodiment of the present invention, the hearing-aid diagnosis and treatment unit comprises a hearing diagnosis and auditory assessment module and a hearing-aid fitting module; the hearing diagnosis and auditory assessment module detects the real hearing level of an amblyacousia patient and makes an hearing assessment through an objective hearing diagnosis and auditory assessment module (for example, one or more of the sound impedance OAE test, auditory brainstem response (ABR) test, auditory steady state response (ASSR) test), and\or a subjective hearing diagnosis and auditory assessment module (for example, pure tone hearing test) in integral seam-less connection with the all-digital audio and tone signal synthesis generator, so as to directly output hearing diagnosis and auditory assessment results to the hearing-aid fitting module or the printer, and the hearing-aid fitting module correspondingly programs and fits a hearing aid according to the actual hearing and auditory situations of the applicable patient.

In a preferably embodiment of the present invention, the hearing diagnosis and auditory assessment module may be a remote hearing diagnosis and auditory assessment module, realizing remote control and data transmission or storage through wireless communication connection with the wireless transmission module disposed in the hearing diagnosis and treatment device.

In a preferably embodiment of the present invention, the tinnitus diagnosis and treatment unit comprises a tinnitus diagnosis module and a tinnitus therapeutic instrument configuration module. The tinnitus diagnosis module stimulates individual tinnitus sounds of a tinnitus patient through the all-digital audio and tone signal synthesis generator and various tinnitus sounds meeting the needs of chemically clinic diagnosis so as to directly output a diagnosis result or a tinnitus treatment prescription to a tinnitus therapeutic instrument configuration module or a printer. The tinnitus therapeutic instrument configuration module correspondingly generates an individual tinnitus diagnostic unit applicable to the patient; the tinnitus treatment prescription is one or two of a tinnitus masking therapy and tinnitus retraining therapy; the tinnitus diagnostic units are classified into two major types, one with tinnitus but no hearing loss and the other with both tinnitus and hearing loss.

In a preferably embodiment of the present invention, the tinnitus diagnosis module may be a remote tinnitus diagnosis module, realizing remote control and data transmission or storage through wireless communication connection with the wireless transmission module disposed in the hearing diagnosis and treatment device.

In a preferably embodiment of the present invention, the hearing-aid diagnosis and treatment unit comprises a haring diagnosis and auditory assessment module and a hearing-aid fitting module. The hearing-aid diagnosis and treatment unit detects the real hearing level of an amblyacousia patient and makes a hearing assessment through the all-digital audio and tone signal synthesis generator so as to directly output a diagnosis result to the hearing-aid fitting module or printer. The tinnitus diagnosis and treatment unit comprises a tinnitus diagnosis module and a tinnitus therapeutic instrument configuration module; the hearing diagnosis and treatment device comprehensively generates a tinnitus treatment hearing aid applicable to the amblyacousia patient through the hearing-aid fitting module and the tinnitus diagnostic unit fitting module. The hearing-aid diagnosis and treatment unit and the tinnitus diagnosis and treatment unit can be operated independently and synchronously.

In a preferably embodiment of the present invention, the tinnitus diagnosis module may be a remote tinnitus diagnosis module, and the hearing-aid diagnosis module may also be a remote hearing-aid diagnosis module, realizing remote control and data transmission or storage through wireless communication connection with the wireless transmission module disposed in the hearing diagnosis and treatment device.

The present invention has the following beneficial effects: The hearing diagnosis and treatment device of the present invention integrates functions of hearing diagnosis and auditory assessment, hearing aid test and fitting, tinnitus diagnosis and tinnitus treatment, shares one all-digital audio and tone signal synthesis generator, is simplified and saves cost. The hearing test is directly implemented in the environment where the hearing aid is used, capable of avoiding the situation that the pure tone hearing test in the environment of the traditional mute room or sound isolating room cannot accurately reflect the nonlinear relationship of the hearing in the noisy environment. The hearing test module records the hearing test result of a patient and outputs a test result directly to a hearing-aid programming and fitting module so as to avoid errors caused by the traditional manual input, and the hearing-aid programming and fitting module fits and adjusts the hearing compensation prescription of the hearing aid. Meanwhile, the hearing diagnosis and treatment device can also diagnose and treat tinnitus, provide individual and specific treatment to the amblyacousia patients, the tinnitus patients and the patients suffering both amblyacousia and tinnitus, applicable to various auditory handicapped groups from children to the old. Moreover, the integrated hearing diagnosis and treatment device provided by the present invention is portable, operable by just one technician, convenient and widely applicable, and can be used at various medical and living sites such as hospitals, community clinics, houses or out-patient departments.

### BRIEF DESCRIPTION OF THE DRAWINGS

Figure 1 is a structure diagram of a hearing diagnosis and treatment device in a preferable embodiment of the present invention.
Figure 2 is a structural view of a hearing-aid diagnosis and treatment unit of a hearing diagnosis and treatment device in a preferable embodiment of the present invention.
Figure 3 is a structural view of a tinnitus diagnosis and treatment unit of a hearing diagnosis and treatment device in a preferable embodiment of the present invention.

### DETAILED DESCRIPTION OF THE INVENTION

The preferable embodiments are described in detail with reference to the attached drawings such that those skilled in this field can more easily understand the advantages and characteristics of the present invention to more clearly define the protective scope of the present invention.

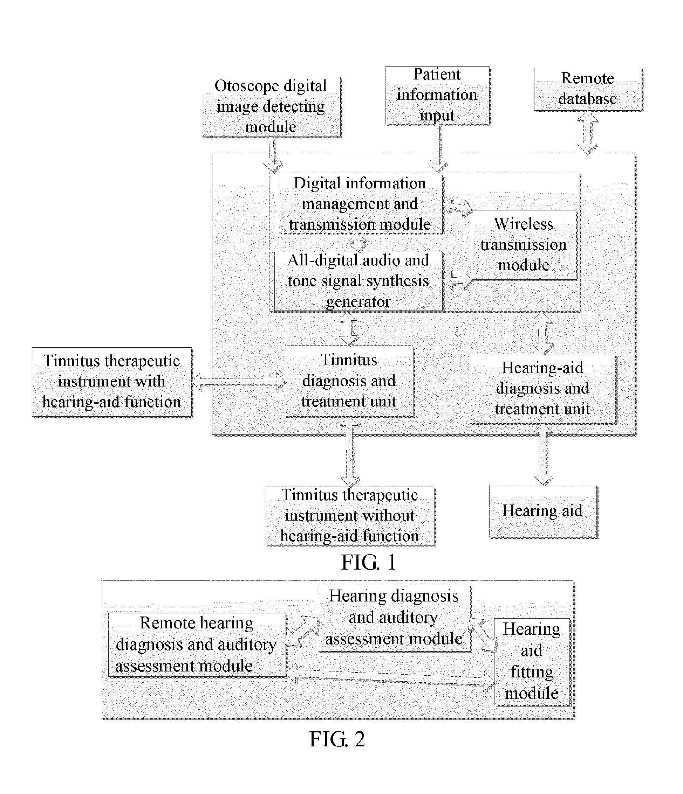

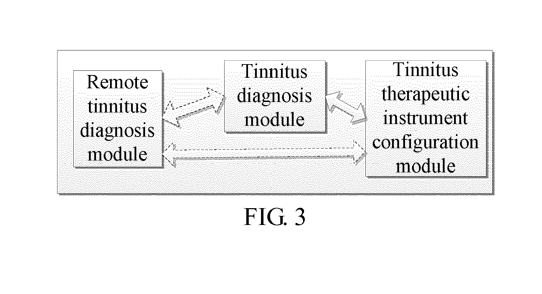

Refer to Figures 1, 2, and 3. This embodiment of the present invention provides a hearing diagnosis and treatment device. The hearing diagnosis and treatment device can be externally connected with a hearing aid and a tinnitus treatment device and is connected with a remote database in wireless communication way. The hearing diagnosis and treatment device of the present invention comprises an all-digital audio and tone signal synthesis generator, a digital information management and transmission module, a tinnitus diagnosis and treatment unit and a hearing-aid diagnosis and treatment unit; the tinnitus diagnosis and treatment unit comprises a tinnitus diagnosis module and a tinnitus therapeutic instrument configuration module and also comprises a remote tinnitus diagnosis module. The hearing-aid diagnosis and treatment module comprises a hearing diagnosis and auditory assessment module, a hearing-aid fitting module and comprises a remote hearing diagnosis and auditory assessment module.

The patient information is input to the digital information management and transmission module. An external or built-in an otoscope digital image detecting module detects the ears of the patient through mirroring generates image pictures of the auditory meatus and tympanic membrane and transmits the same to the digital information management and transmission module for storage. After a patient wears the hearing aid, the hearing diagnosis and treatment and auditory assessment module in the hearing diagnosis unit detects the actual hearing situation of the patient according to the all-digital audio and tone signal synthesis generator, records the hearing test and auditory assessment result of the patient and outputs a pure tone hearing diagram. The pure tone hearing diagram can be respectively transmitted to the digital information management and transmission module, the remote hearing-aid diagnosis module and the hearing-aid fitting module. The hearing-aid fitting module performs fitting and programming according to the hearing test result or the pure tone hearing diagram, and the hearing-aid fitting module implements the hearing compensation prescription for fitting and adjusting the hearing aid. The tinnitus diagnosis module in the tinnitus diagnosis and treatment unit stimulates or reduces the tinnitus sound of the tinnitus patient according to the all-digital audio and tone signal synthesis generator, records the tinnitus diagnosis result of the patient, transmits the tinnitus diagnosis result to the information management and transmission module, the remote tinnitus diagnosis module and the tinnitus therapeutic instrument configuration module. The tinnitus therapeutic instrument configuration module performs the tinnitus masking or tinnitus acclimatization treatment prescription for fitting and adjusting the hearing aid and the tinnitus therapeutic instrument.

In this embodiment of the present invention, the all-digital audio and tone signal synthesis generator can generate acoustic digital signals from single tone to compound tone such as the pure tone and\or noises and \or compound sound and\or simulated animal sound and\or simulated human speech, and can randomly adjust the frequency and\ or loudness and\or tone and \or melody and \or rhythm. The all-digital audio and tone signal synthesis generator is in integrated, seamless connection with an objective hearing detection module (for example, one or more of the sound impedance OAE test, auditory brainstem response (ABR) test, auditory steady state response (ASSR) test), and\or a subjective hearing detection module (for example, pure tone test), so as to detect the real hearing situation of the patient and assess the auditory sense of the patient.

The digital information management and transmission module is mainly used for patient information input, creation and storage of medical records, information management and transmission, and storage of the all-digital diagnosis and treatment information. Wherein, the stored all-digital diagnosis and treatment information comprises image pictures of the auditory meatus and tympanic membrane, hearing-aid diagnosis information, tinnitus diagnosis information, programming and fitting information of the hearing compensation hearing aid, and the tinnitus treatment prescription information.

The USB interface of the otoscope digital image detecting module is connected with an external digital video otoscope. The otoscope digital image detecting module has a high-brightness LED lamp, a hard or soft probe capable of extending into the auditory meatus, and amplify the images by 10X to 200X. The otoscope digital image detecting module has advantages of high-speed photographing, high definition, high frame speed, and high pixel; the digital photos can be stored as image documents in the formats of JPG, JPEG, etc.

The hearing diagnosis and treatment device is externally connected with the hearing aid, the tinnitus therapeutic instrument and the remote database. The remote database and the digital information management and transmission module perform data transmission, exchange and storage through a wireless transmission module.

The output of the hearing-aid test result may be but is not limited to the pure tone hearing diagram, including the hearing threshold and the uncomfortable threshold. The pure tone hearing diagram can be stored as picture document (pdf) and audio signal to be output and printed and be in seamless connection with the hearing-aid fitting module.

The stimulating sound needed by the hearing diagnosis and auditory assessment module is an intermittent or continuous single tone or multi-tone sound signal, including variables such as the frequency, loudness and waveform. On condition of a given frequency, the sound intensity adjusting modes of the hearing diagnosis and auditory assessment module include static manual adjustment, dynamic adding, constant sound intensity or gradual increase of the sound intensity, dynamic variation speed, and intermittent or continuous sound. The patient can also conveniently operate the dynamic added sound intensity scanning test. After wearing the earphone, the patient can operate an external or a built in user controller, including but not limited to a Joystick, press the button or mouse, and quickly and accurately record the hearing threshold and uncomfortable threshold. The hearing test can be implemented in any set frequency and sound pressure level scope, for example, 150Hz- 8500Hz, 0dB - 120dB.

The hearing aid amplifies the sounds, which the hearing-impaired patients cannot hear according to the demands, and uses the residual hearing to send the sounds to the auditory center of the brain, and then the patient can hear the sounds. A microphone converts a sound wave into an electric signal. A digital signal processor (DSP) amplifies and optimizes the electric signal, and then a telephone receiver converts the electric signal into a sound signal and transmits the same into the ear. The hearing-aid fitting module performs fitting and programming according to the hearing test result and pure tone hearing diagram of the patient.

In the present invention, the pure tone hearing diagram received by the hearing-aid fitting module may be directly input from the hearing test and auditory assessment module, and may be manually input external pure tone hearing diagram. The hearing-aid fitting module is built in with a plurality of hearing compensation prescription formulas, convenient to select to the hearing environment (various noise environment, sound source position, working and living environments, etc.). The hearing-aid fitting module is also built in a hearing meter, which can perform a secondary hearing test according to individual needs and dynamically coupled with the fitting and adjustment to adjust the multi-band output power and gain.

In this embodiment of the present invention, the remote hearing-aid diagnosis module and the remote tinnitus diagnosis module remotely adjust the fitting programming signal of the hearing aid or the programming signal of the tinnitus therapeutic instrument, transmitting the signals in a wireless way through an open platform of a mobile phone. The hearing aid or tinnitus therapeutic instrument is equipped with a wireless transmission module GPRS, directly realizing machine-machine dialog and signal transmission with the hearing diagnosis and treatment device of the present invention.

In this embodiment of the present invention, the tinnitus therapeutic instrument confirmation module is built in with a plurality of formulas of the tinnitus masking or tinnitus acclimatization treatment prescription; the hearing aid is also provided with a wireless transmission module GPRS for communication connection with the remote hearing-aid diagnosis module and the remote tinnitus diagnosis module. The tinnitus therapeutic instrument confirmation module fine tunes the corresponding frequency and loudness according to the individual test of the patient to output the tinnitus sound signal meeting the individual needs of the patient.

The two physical methods, namely tinnitus masking and tinnitus acclimatization, basically share the same principle: re-train or re-code the nerve system to reduce the excitation of the central nerve system, increase restraint on the central nerve system, cut off the vicious circle of tinnitus and bad emotions, promote the patient to be adapted to the tinnitus, not to sense the tinnitus or reduce the sense to the tinnitus, thus fulfilling the aim of mitigation or treatment. Tinnitus treatment prescription is location or audio digital expression of the tinnitus sound signal, containing compound parameters (including tone, rhythm, melody, etc.) and basic parameters (including central frequency, bandwidth and loudness), can be stored as the audio signal diagrams in the format of pdf to be printed or stored; or the encoder converts the time domain waveform signal into the frequency domain signal to directly output the non-compressed or compressed frequency domain audio, including but not limited to .wav, .mp3, .mp5, .cad, .ram, .wma, .mid, etc., then the audio is downloaded to the tinnitus therapeutic instrument based on the audio signal player and then decoded such that the patient wearing the tinnitus therapeutic instrument can hear; or, the tinnitus treatment prescription can be seamlessly input into the hearing-aid fitting module to provide the tinnitus patient with an individual tinnitus treating hearing aid with hearing compensation and tinnitus treatment functions through programming and debugging.

The fitting of the hearing aid can perform debugging on one ear or two ears at the same time; two-ear balance debugging achieves better effects on the majority of the patients with hearing impairment, in particular to the tinnitus patients; the individual debugging can be implemented after the hearing aids of the left and right ears are mutually copied.

The hearing diagnosis and treatment device of the present invention is small sized and portable, has high resistance to electromagnetic interference in the environment, is well grounded and shields the electrostatic and low-frequency AC electric fields. The housing of the hearing diagnosis and treatment device and the shell of the evoked potential machine in the device form dual-layer shielding, both using high magnetic conductive materials (such as iron, silicon steel sheet, permalloy, etc.) to form a low-magnetic-resistance passage so as to shield the magnetostatic and low-frequency AC magnetic fields. Reflection and attenuation are employed to isolate the electromagnetic field coupling, and the dual-layer shielding housing made from materials with high magnetic conductivity and high electric conductivity has a large absorption loss and prevents electromagnetic leakage from seams. The outside shielding layer of the hearing diagnosis and treatment device is provided with a honeycomb-shaped ventilating board to prevent leakage of the electromagnetic wave; the shell of the evoked potential machine is lined with a silicone rubber liner, and the liner contains highly electrically conductive metal (such as stainless steel, etc.) meshes which account for 70%-80% of the volume. The low-impedance and high-sensitivity electrode has a silver core externally wrapped with a multi-phase alloy MP35N composite electrode material; and the evoked potential machine shares the pure tone hearing test module and the display module.

The hearing diagnosis and treatment device of the present invention integrates functions of hearing diagnosis and auditory assessment, hearing aid test and fitting, tinnitus diagnosis and treatment, is simplified and saves cost. The hearing diagnosis and auditory assessment is directly implemented in the environment where the hearing aid is used, capable of avoiding the situation that the pure tone hearing test in the environment of the traditional mute room or sound isolating room cannot accurately reflect the nonlinear relationship of the hearing in the noisy environment. The hearing diagnosis and auditory assessment module records the hearing test result of a patient and outputs a test result directly to a hearing-aid programming and fitting module so as to avoid errors caused by the traditional manual input, and the hearing-aid programming and fitting module fits and adjusts the hearing compensation prescription of the hearing aid. Meanwhile, the hearing diagnosis and treatment device can also diagnose and treat tinnitus, provide individual and specific treatment to the amblyacousia patients, the tinnitus patients and the patients suffering both amblyacousia and tinnitus, applicable to various auditory handicapped groups from children to the old. Moreover, the hearing diagnosis and treatment device provided by the present invention is portable, operable by just one technician, convenient and widely applicable, and can be used at various medical and living sites such as hospitals, community clinics, houses or out-patient departments.

The above are only some embodiments of the present invention and shall not be regarded as limit to the present invention. Any equivalent structure or equivalent flow modifications made based on the description and attached drawings of the present invention, or director or indirect application to other related fields, shall fall within the protective scope of the present invention.

## Claims

1. A hearing diagnosis and treatment device, **characterized by** comprising an all-digital audio and tone signal synthesis generator for generating various audio and tone signals meeting hearing diagnosis and treatment conditions; a digital information management and transmission module connected with the all-digital audio and tone signal synthesis generator; and also comprising a tinnitus diagnosis and treatment unit and a hearing-aid diagnosis and treatment unit which are connected with the all-digital audio and tone signal synthesis generator.

2. The hearing diagnosis and treatment device according to claim 1, wherein the all-digital audio and tone signal synthesis generator can generate acoustic digital signals from single tone to compound tone such as the pure tone and \or noises and\or compound sound and \or simulated animal sound and \or simulated human speech, and can randomly adjust the frequency and\ or loudness and\or tone and \or melody and \or rhythm, the tinnitus diagnosis and treatment unit and the hearing-aid diagnosis and treatment unit share the same all-digital audio and tone signal synthesis generator.

3. The hearing diagnosis and treatment device according to claim 1, wherein the hearing diagnosis and treatment device also comprises an otoscope digital image detecting module for detecting the auditory meatus and tympanic membrane of a patient and outputting detection results in digital and image form to the digital information management and transmission module or a printer.

4. The hearing diagnosis and treatment device according to claim 1, wherein the hearing diagnosis and treatment device is internally provided with a wireless transmission module, capable of exchanging information such as the digital images of the auditory meatus and tympanic membrane, the test result and treatment prescriptions of the tinnitus diagnosis and treatment unit, the hearing test and auditory assessment results and hearing compensation and optimizing prescriptions of the hearing-aid diagnosis and treatment unit in the digital information management and transmission module with the information in a remote database or storing the information.

5. The hearing diagnosis and treatment device according to any one of claims 1-4, wherein the hearing-aid diagnosis and treatment unit comprises a hearing diagnosis and auditory assessment module and a hearing-aid fitting module; the hearing diagnosis and auditory assessment module detects the real hearing level of an amblyacousia patient and makes an hearing assessment through an objective hearing diagnosis and\or subjective hearing diagnosis module in integral seam-less connection with the all-digital audio and tone signal synthesis generator and an auditory assessment module, so as to directly output hearing diagnosis and auditory assessment results to the hearing-aid fitting module or printer, and the hearing-aid fitting module correspondingly program and fit a hearing aid according to the actual hearing and auditory situations of the applicable patient.

6. The hearing diagnosis and treatment device according to claim 5, wherein the hearing-aid diagnosis and treatment module may be a remote hearing diagnosis and auditory assessment module, realizing remote control and data transmission or storage through wireless communication connection with the wireless transmission module disposed in the hearing diagnosis and treatment device.

7. The hearing diagnosis and treatment device according to any one of claims 1-4, wherein the tinnitus diagnosis and treatment unit comprises a tinnitus diagnosis module and a tinnitus therapeutic instrument configuration module, the tinnitus diagnosis module stimulates individual tinnitus sounds of a tinnitus patient through the all-digital audio and tone signal synthesis generator and various tinnitus sounds meeting the needs of chemically clinic diagnosis so as to directly output a tinnitus diagnosis result or a tinnitus treatment prescription to a tinnitus therapeutic instrument configuration module or a printer, the tinnitus therapeutic instrument configuration module correspondingly generates an individual tinnitus diagnostic unit applicable to the patient; the tinnitus treatment prescription is one or two of a tinnitus masking therapy and tinnitus retraining therapy, the tinnitus diagnostic units are classified into two major types, one with tinnitus but no hearing loss and the other with both tinnitus and hearing loss.

8. The hearing diagnosis and treatment device according to claim 7, wherein the tinnitus diagnosis module may be a remote tinnitus diagnosis module, realizing remote control and data transmission and storage through wireless communication connection with the wireless transmission module disposed in the hearing diagnosis and treatment device.

9. The hearing diagnosis and treatment device according to any one of claims 1-4, wherein the hearing-aid diagnosis and treatment unit comprises a haring diagnosis and auditory assessment module and a hearing-aid fitting module, the hearing-aid diagnosis and treatment unit detects the real hearing level of an amblyacousia patient and makes a hearing assessment through the all-digital audio and tone signal synthesis generator so as to directly output a diagnosis result to the hearing-aid fitting module or printer, the tinnitus diagnosis and treatment unit comprises a tinnitus diagnosis module and a tinnitus therapeutic instrument configuration module; the hearing diagnosis and treatment device comprehensively generates a tinnitus treatment hearing aid applicable to a patient with hearing loss and tinnitus through the hearing-aid fitting module and the tinnitus diagnostic unit fitting module, the hearing-aid diagnosis and treatment unit and the tinnitus diagnosis and treatment unit can be operated independently and synchronously.

10. The hearing diagnosis and treatment device according to claim 9, wherein the tinnitus diagnosis module may be a remote tinnitus diagnosis module, and the hearing-aid diagnosis module may also be a remote hearing-aid diagnosis module, realizing remote control and data transmission and storage through wireless communication connection with the wireless transmission module disposed in the hearing diagnosis and treatment device.
